# EUROPEAN PATENT APPLICATION

(11) **EP 3 699 285 A1**
(43) Date of publication of application: **26.08.2020**
(21) Application number: 18867462.6
(22) Date of filing: 04.09.2018
(51) Int. Cl.: C12N 15/85, A01K 67/027

(54) **USE OF NOK GENE AND EXPRESSION PRODUCT THEREOF IN CONSTRUCTION OF ANIMAL MODEL OF CHRONIC B LYMPHOCYTIC LEUKEMIA**

(30) Priority: 16.10.2017 CN 201710958353; 14.12.2017 CN 201711337232
(71) Applicant: Institute of Basic Medical Sciences, Chinese Academy of Medical Sciences, Beijing 100005 (CN)
(72) Inventor: LIU, Li, Beijing 100005 (CN)
(74) Representative: Sonzogni, Laura Gabriella
(86) International application number: PCT/CN2018/103982
(87) International publication number: WO 2019/076154

(57) **Abstract**

Provided is the use of a NOK gene and an expression product thereof in construction of an animal model of chronic B lymphocytic leukemia.

Sequence list

<110>  Institute of Basic Medical Sciences, Chinese Academy of Medical
Sciences

<120>  Use of NOK gene and expression product thereof in construction of
animal model of chronic B lymphocytic leukemia

<130>  370402CG-370304

<160>  10

<170>  SIPOSequenceListing 1.0

<210>  1
<211>  30
<212>  DNA
<213>  Artificial Sequence

<400>  1
tggcccgcct ggcattatgc ccagtacatg                                        30

<210>  2
<211>  30
<212>  DNA
<213>  Artificial Sequence

<400>  2
agccacagga tgaccccaag aaggatgagg                                        30

<210>  3
<211>  30
<212>  DNA
<213>  Artificial Sequence

<400>  3
tcctgaagtc cctcctacca gcatcctaga                                        30

<210>  4
<211>  30
<212>  DNA
<213>  Artificial Sequence

<400>  4
tcttcccaat cctccccctt gctgtcctgc                                        30

<210>  5
<211>  17
<212>  DNA
<213>  Artificial Sequence

<400>  5
atcggaaagc aggtcct                                                      17

<210>  6
<211>  17
<212>  DNA
<213>  Artificial Sequence

<400>  6
aagaggctct ccactct                                                      17

<210>  7

<211>  20
<212>  DNA
<213>  Artificial Sequence

<400>  7
taatcattgt tccggctctc                                                   20

<210>  8
<211>  20
<212>  DNA
<213>  Artificial Sequence

<400>  8
ccagatgaca aagtttggga                                                   20

<210>  9
<211>  20
<212>  DNA
<213>  Artificial Sequence

<400>  9
cacactgtgc ccatctacga                                                   20

<210>  10
<211>  20
<212>  DNA
<213>  Artificial Sequence

<400>  10
ctgcttgctg atccacatct                                                   20

## Description

### FIELD OF THE INVENTION

The present application relates to the fields of biology and medicine. It especially relates to the use of NOK gene in the construction of animal model of B-cell chronic lymphocytic leukemia.

### BACKGROUND OF THE INVENTION

B-cell chronic lymphocytic leukemia (B-CLL) is the commonest type of leukemia among western population, and is mainly caused by the accumulation and clonal expansion of CD5⁺/CD19⁺ B lymphocytes (1-4). The incidence rates of B-CLL are higher in both US and UK with 3.5 and 6.15 cases per 100,000 individuals, respectively (2). The incidence rate increases with age. The peak of incidence is around the mean age of 65 years (4). The progression of B-CLL can be either indolent or aggressive. The majority of patients usually survive for decades after diagnosis, and finally die of leukaemia. However, the disease could also progress into an aggressive form. Although patients have been provided with intensive treatments, the disease still leads to rapid death of patients.

B-CLL is a heterogeneous disease that differs dramatically, not only in its severity of clinical manifestation, but also in the mutation status and signaling events associated with B cell receptor (BCR) (1). B-CLL can be classified into mutated B-CLL (M-CLL) and unmutated B-CLL (U-CLL), according to the mutation status of immunoglobin heavy chain variable region (IGHV) (5, 6). The presence of M-CLL indicates that some clonal founder cells of CLL may behave like normal B lymphocytes and undergo somatic hyper-mutation in IGHV gene upon encountering antigen inside or outside the germinal center (7). U-CLL can be regarded as the expansion of a leukemia clone that was repetitively stimulated by microbes or autoantigens. Regardless of the mutation status of IGHV, the gene expression profile of M-CLL and U-CLL cells is similar to that of memory B cells (8, 9). In the case of unmutated U-CLL, B cells are usually signaling-competent upon antigen ligation with surface IgM (10); and in most cases they highly express CD38⁺ and/or ZAP-70⁺ molecules associated with fatal outcome (11-15). In contrast, in the case of mutated M-CLL cells, mutation in IGHV gene may eventually block BCR mediated signaling cascade which is induced by engagement of antigen with BCR (10, 16). This signaling blockade might be mainly due to either the inability or high avidity of antigen recognition, since IGHV gene has been mutated. M-CLL cells with few expression of CD38⁺ and few expression of ZAP-70⁺ are usually indolent (17). Despite intensive research on the pathogenesis of B-CLL, the origin of B-CLL cells remains uncertain. Ethnic differences and genetic lesions may have great impacts on the development of B-cell chronic leukemia (B-CLL) (18-20). Results of epidemiological studies pointed out that the environmental factors might not be predominant for B-CLL development, since the incidence rates of the Asian immigrants to the Western countries are still extremely low (19). First degree relative analysis indicated that familial relevance is high for B-CLL, implying that some susceptive genes might play some key roles in the early-onset of B-CLL (21, 22). Molecular analysis on large cohort B-CLL patient samples revealed several genomic abnormalities, such as deletion on chromosome 13q14 (accounted for about 55% of cases) (23, 24), and trisomy of chromosome 12 (accounted for about 16% to 35% of cases) (25-27).

### SUMMARY OF THE INVENTION

*NOK* is a novel oncogene derived from human chromosome 12 of which trisomy is associated with B-CLL. NOK could induce cellular transformation as well as tumorigenesis and metastasis in animal (28, 29).

The inventor has studied the function of NOK gene at the whole animal level by using NOK-tg mice, and found that NOK-tg mice can spontaneously develop B-lymphocyte disorder that is extremely resemble to human B-CLL. Immunophenotypic analysis strongly indicated that NOK may transform primitive stem cells towards B-CLL in bone marrow microenvironment.

According to such facts, the present disclosure provides the use of NOK gene or its expression product in the construction of animal model of chronic lymphocytic leukemia.

In some embodiments, the chronic lymphocytic leukemia is chronic B lymphocytic leukemia.

In some specific embodiments, chronic B-lymphocytic leukemia includes, but is not limited to: mutant chronic B-lymphocytic leukemia (M-CLL), unmutated chronic B-lymphocytic leukemia (U-CLL).

In some embodiments, the animal is a mammal other than human, such as, but not limited to, mouse, rat, guinea pig, rabbit, bovidae, caprid, equidae, camelid, suidae, canidae, felidae, monkey or ape. In some specific embodiments, the animal is mouse.

In some embodiments, the animal model shows any one symptom or combination thereof selected from the group consisting of: weight loss, fatigue, itching, bleeding points on skin, organ enlargement, invasion of tumor cells into organ. In some specific embodiments, the organ is selected from: liver, spleen, kidney, lung, lymph node, peripheral blood, bone marrow, or combination thereof.

In some embodiments, the NOK is a human NOK. The nucleotide sequence of human NOK can be obtained in a public database, and the Genbank accession number is KP729000.1. The nucleotide sequences of NOK for other species are also available in public databases.

In some embodiments, the expression product of NOK gene refers to any form of the NOK gene at any stage in its life cycle, such as but not limited to: mRNA of NOK or its complementary sequence, cDNA of NOK or its complementary sequence, mature protein, precursor protein of NOK, or a fragment of any of the above.

According to some embodiments, a method for constructing animal model of chronic lymphocytic leukemia is provided, including:
1) providing an expression vector expressing human NOK,
2) introducing the expression vector into oocyte of animal;
3) transferring the oocyte injected with the expression vector to surrogate animal for growth;
4) NOK transgenic animal(s) is/are produced by the surrogate animal.

In some embodiments, the expression vector is introduced into an oocyte of an animal by microinjection.

In some embodiments, the chronic lymphocytic leukemia is chronic B lymphocytic leukemia. Preferably, the chronic B lymphocytic leukemia is a mutant chronic B lymphocytic leukemia, or an unmutated chronic B lymphocytic leukemia.

In some embodiments, an animal model suitable for use in the methods of the present application is mammal other than human. In some embodiments, the animal is selected from the group consisting of mouse, rat, guinea pig, rabbit, bovidae, caprid, equidae, camelid, suidae, canidae, felidae, monkey, ape. In some embodiments, the animal is mouse.

According to some embodiments, a method for constructing a mouse animal model of chronic lymphocytic leukemia is provided, comprising: 1) providing an expression vector expressing human NOK; 2) introducing the expression vector into mouse oocyte, preferably introduced by microinjection; 3) the oocyte injected with the expression vector is transferred to a surrogate mouse for growth; 4) NOK transgenic mouse is obtained from the surrogate mouse.

### DESCRIPTION OF THE DRAWINGS

**Figure 1****.** Schematic diagram showing the *NOK* expression cassette that was used for the injection of fertilized eggs. HA tag was fused to the 3' end of *NOK* cDNA. Two pairs of primers (PI & P2 and P3 & P4) were designed for genomic PCR analysis.
**Figure 2****.** Genomic PCR analysis on the mouse tail DNAs from *NOK* transgenic mice.
**Figure 3****.** Tissue distributions of human and mouse *NOK* mRNAs detected by RT-PCR analysis. Total RNA from tissue cells were isolated from different tissues of *NOK* transgenic (*NOK-*tg) mouse. The expression of β-actin was served as internal control.
**Figure 4****.** Tissue distributions of mouse *NOK* mRNAs detected by RT-PCR analysis. Total RNA from tissue cells were isolated from different tissues of wild type (WT) mouse. The expression of β-actin was served as internal control.
**Figure 5****.** The expression profile of NOK protein was detected by Western blot analysis. Suspensions of total cellular proteins were prepared from the selected tissues of either wild type or *NOK-tg* mice. The equal amounts of protein samples were resolved on gel, transferred onto membrane and finally probed with rabbit anti-NOK antibodies. The protein loading was monitored with the expression level of β-actin.
**Figure 6A and 6B****.** The progressive lymphadenectasis in *NOK-tg* mouse. Lymphadenectasis in *NOK-tg* mouse was usually initiated at one side of the body. Photographs were taken at the 41^{st} and 44^{th} weeks after birth.
**Figure 7****.** The enlarged lymph nodes in neck of *NOK-tg* mouse.
**Figure 8A and 8B****.** Wright's staining on the smear samples of peripheral blood from *NOK-tg* and WT mice.
**Figure 9****.** Peroxidase (Pox) staining on the smear samples of peripheral blood of *NOK-tg.* Arrow indicates Pox positive cell. Stars indicate the Pox negative leukemic cells.
**Figure 10A to 10F****.** Pathological analysis of major tissues in *NOK* transgenic mice. Haematoxylin & Eosin (HE) staining was conducted on the tissue sections made from kidney (A), lung (B), liver (C), and small intestine (D) of *NOK* transgenic mouse (Magnification × 100). Immunohistochemistical staining was performed on the tissue sections prepared from lung (E) and liver (F) of *NOK-tg* transgenic mouse (Magnification × 200). The enlarged window of the selected field was inserted into the right corner of each photo. T = tumor, and N = normal.
**Figure 11****.** Immunophenotypic characterization of *NOK-tg* mice. CD3⁻/CD5⁺ lymphocyte population was significantly expanded in the peripheral lymphoid organs of *NOK-tg* mice with Lymph node metastasis. PBMC and lymph node cells derived from WT and *NOK-tg* mice were doubly stained with both FITC anti-CD3 and PE-Cy5 anti-CD5, and then subjected to flow cytometric analysis. The data represents one of the four independent tests with similar results.
**Figure 12****.** Analysis of CD5 and CD19 expressions by flow cytometry on single cell suspensions prepared from PBMCs and lymph nodes (LN). The single cell suspensions of PBMCs and LN were made from WT and *NOK-tg* mice. Two color flow cytometric analysis using PE-Cy5 anti-CD5 and Biotin anti-CD19 antibodies was performed.
**Figure 13****.** Quantitation of the percentage of CD5⁺CD19⁺ population, at least three to four independent tests were performed. Statistical analysis was performed using student's t test.

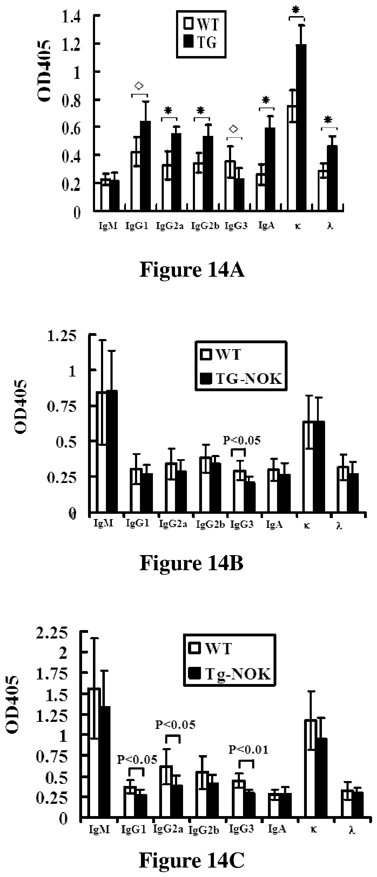

**Figure 14A****.** Analysis of immunoglobin production and antigen specific humoral response. The relative serum levels of immunoglobins and their switching variants were analyzed by ELISA test. Sera were collected from 6 *NOK-tg* (TG) and 6 age-matched control mice (WT), and diluted into 1:1,000. A 96-well ELISA plate was first coated with fixed amount of goat anti-mouse Ig as capture antibodies. The diluted sera were then added into each well. The reaction mixtures were assayed with standard ELISA kit. The results were recorded with a spectrometer at OD405. Hollow diamond represents *p*<0.05, while solid black star indicates *p*<0.01.
**Figure 14B and 14C****.** ELISA analysis on the relative levels of SRBC specific immunoglobins and their switching variants, before and after one-week SRBC immunization. Sera were collected from 6 *NOK-tg* and 6 age-matched control mice, and diluted 1:1,000 with PBS. After coating 96 well plate with SRBCs, 100µl of the diluted serum was added to each well. The reaction mixtures were assayed with standard ELISA kit.
**Figure 15A to 15F****.** Evaluation of antigen specific Ig isotypes and isotype switching variants after primary (1°) and secondary (2°) immunizations with SRBCs. Sera were collected from 6 *NOK-tg* and 6 age-matched control mice at each time point, and diluted 1:1,000 with PBS. The 96-well plate was coated with SRBC before the addition of the diluted serum. The reaction mixtures were assayed with standard ELISA kit. The solid black star indicates *p*<0.01.

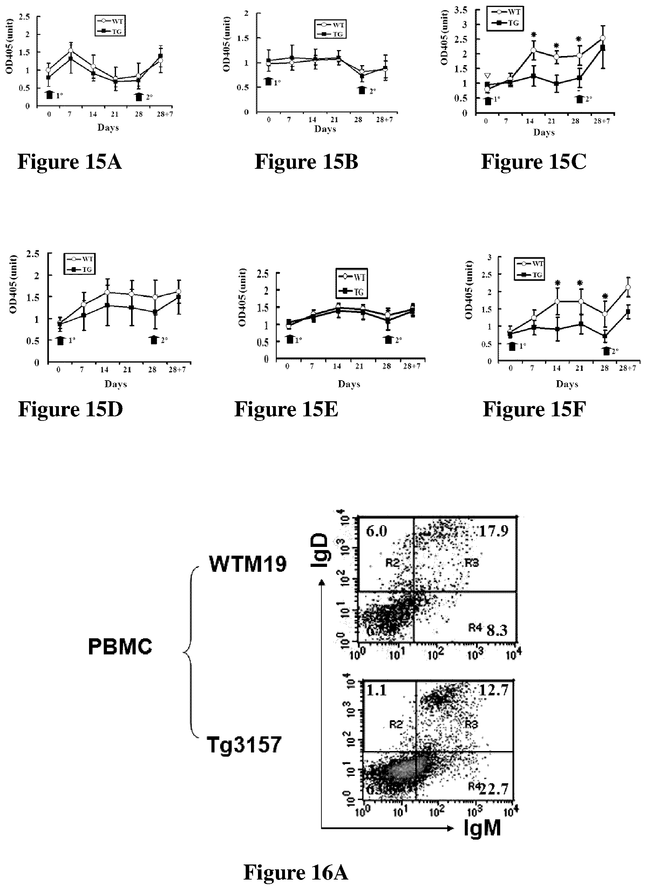

**Figure 16A to 16F****.** The memory phenotypes of B lymphocyte cells in the peripheral blood and lymphoid tissues. Four-color flow cytometric analysis on PBMCs (16A, 16B), spleens (16C, 16D) and lymph nodes (16E, 16F) was performed using PE-Cy7 anti-IgM, Biotin anti-IgD, PE anti-CD27 and FITC anti-CD23 antibodies. The reaction mixtures were first assayed for the expressions of IgM and IgD, and then the gated cells were selected for further analysis on CD27 and CD23 expressions. The results are the representation of at least three independent tests for WT mice and five independent tests for *NOK-tg* mice.
**Figure 17****.** Identification of the expanded population of hematopoietic stem cells in *NOK-tg* mice. Two color flow cytometric analysis was performed on bone marrow (BM) cells from WT and *NOK-tg* mice that were labeled with PE-Cy7 anti-B220 and Biotin anti-CD43 antibodies. For biotin-conjugated antibody labeling, allophycocyanin-conjugated streptavidin was added to the reaction mixtures. The reaction mixtures were finally analyzed with a flow cytometer.
**Figure 18****.** Quantitation of B220 and CD43 expressions in BM cells from nine *NOK-tg* (solid bar) and nine WT (hollow bar) mice. Statistical test was conducted using student's t test.
**Figure 19A and 19B****.** Three color flow cytometric analysis on BM cells was performed using Biotin anti-lineage (Lin), PE anti-CD43 and FITC anti-Sca-1 antibodies. The reaction mixtures were first assayed for the expressions of lineage Lin and CD43, and then the gated cells were selected for further analysis on Sca-1 expression.
**Figure 20A and 20B****.** Quantitation of the Lin⁺CD43⁺Scal-1⁺ and Lin⁻CD43⁺Scal-1⁺ subpopulations derived from BM cells. Result is mean ± standard deviation from three independent tests.
**Figure 21A and 21B****.** Assessment of c-Kit⁺ cell percentage in lineage negative BM cells of NOK-tg and age-matched control mice. Three color flow cytometric analysis on BM cells was performed using Biotin anti-Lin, PE anti-CD43 and FITC anti-c-kit antibodies. The reaction mixtures were first assayed for the expressions of Lin and CD43, and then the gated cells were selected for further analysis on c-kit expression. The data is the representation of at least three independent tests sharing similar results.
**Figure 22****.** The phenotypes of NOK-tg mice B-CLL model. The enlarged lymph nodes in NOK-tg. The enlarged lymph nodes (indicated by arrow) were usually initiated at one side of the bodies of NOK-tg mice, and were often found in the neck or submaxillary sides.
**Figure 23****.** Comparison of the organ sizes and weights of WT and NOK-tg mice. The main organs such as livers, spleens, kidneys and lungs derived from NOK-tg mice with enlarged lymph nodes (#1761 and #2243) were compared with that from the age-matched control mice (WTM7).
**Figure 24****.** Single cell suspensions were prepared from lymph nodes (LN), peripheral blood mononuclear cells (PBMC), spleen and bone marrow (BM) of WT (WTM10) and NOK-tg mice (Nos. 2880 and 2894), and two-color flow cytometry was performed by using PE Cy7 anti-IgM and PE anti-IgD antibodies. The experimental results represent one of five independent tests sharing similar results.
**Figure 25****.** Cell suspensions prepared from spleen, lymph nodes and BM of WTF5 and NOK-tg (#1455 and #2139) mice were probed with PE-Cy5 anti-CD5 and FITC anti-IgM.
**Figure 26****.** Two color flow cytometric analysis was performed on single cell suspensions prepared from lymph nodes by using PE-Cy5 anti-CD5 and PE anti-CD19 antibodies.
**Figure 27****.** Two color flow cytometric analysis was performed on single cell suspensions prepared from BMs by using of PE anti-IgD and FITC antibodies.
**Figure 28****.** Two color flow cytometric analysis was performed on single cell suspensions prepared from BMs by using PE-Cy5 anti-CD5 and PE anti-CD19 antibodies.

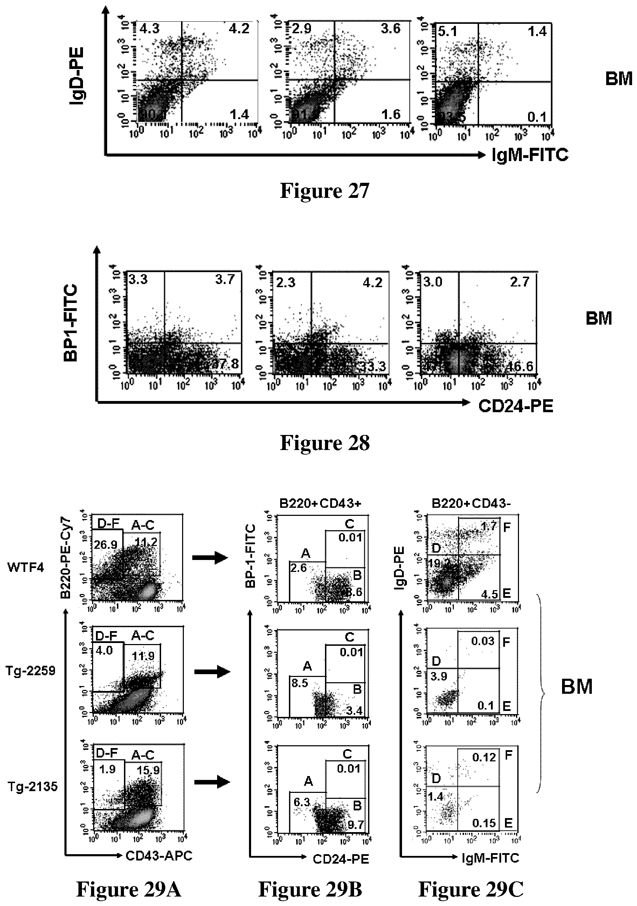

**Figure 29A to 29C****.** Four color flow cytometric analysis shows the distribution of early B lineage in BM cells of WT and NOK-tg mice. BM cells from WT and NOK-tg mice were stained with two groups of antibodies: PE-Cy7 anti-B220, Biotin anti-CD43, FITC anti-BP-1 & PE anti-CD24; and PE-Cy7 anti-B220, Biotin anti-CD43, FITC anti-IgM & PE anti-IgD. Total of six stages (from A to F) were indicated for bone marrow B cell differentiation. For Biotin-conjugated antibody labeling, allophycocyanin-conjugated streptavidin was finally added to the reaction mixture. The experiment result is the representation of at least five independent tests sharing similar results.
**Figure 30****.** The detection of expanded B lymphocyte precursors in the lymph nodes of NOK-tg mice. Two color flow cytometric analysis was performed on lymph node cells of WT and NOK-tg mice by using PE anti-CD43 and FITC anti-CD19.
**Figure 31****.** Quantitation of the CD43⁺/CD19⁺ lymph node cells from WT and NOK-tg mice. Result is mean plus standard deviation from three independent tests.

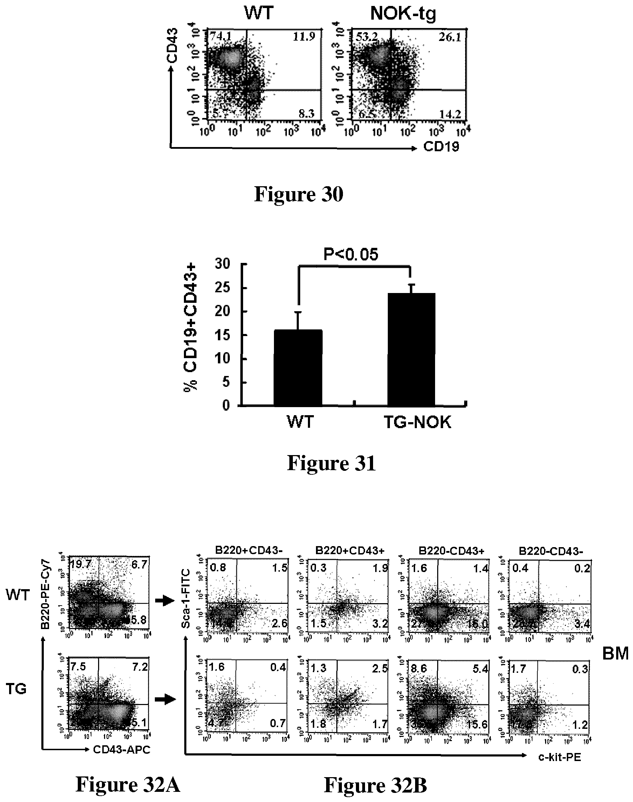

**Figure 32A and 32B****.** Assessment of Sca-1⁺ cell population in the BMs of WT and NOK-tg mice. Four color flow cytometric analysis was performed on BM cells from WT and NOK-tg mice by using PE-Cy7 anti-B220, Biotin anti-CD43, PE anti-c-Kit and FITC anti-Sca-1. The BM cells were first analyzed with PE-Cy7 anti-B220 and Biotin anti-CD43. Then, the gated cells were displayed in a plot with the Log values of FITC anti-Sca-1 versus PE anti-c-Kit as scales respectively. For Biotin-conjugated antibody labeling, allophycocyanin-conjugated streptavidin was finally added to the reaction mixture. Each result is the representation of at least three independent tests sharing similar results.
**Figure 33****.** Two color analysis performed on lineage-depleted BM cells of WT and NOK-tg mice. BM cells isolated from WT and NOK-tg mice were lineage-depleted and subsequently stained with PE anti-CD43 and FITC anti-Sca-1, for Two-color flow cytometric analysis. Each result is the representative of at least three independent tests sharing similar results.

### DETAILED DESCRIPTION OF THE INVENTION

### Generation and identification of NOK transgenic mice

1. *NOK* transgenic mice (NOK-tg mice) were created by standard microinjection technique (52). The mice were maintained and used by following the regulations of Animal Care and Use Policy of the Institute of Basic Medical Sciences of Chinese Academy of Medical Sciences. A 3.9kb DNA fragment containing *NOK* expression cassette driven by CMV promoter was released from pcDNA3.0-NOK-HA (29), by *PvuI* and *SmaI* double digestions, and was gel-purified by DNA gel extract kit (Vigorous, Beijing, China). The reaction product was resuspended to a concentration of 50ng/µL with TE (10mM Tris-HCl, ph7.5 and 1mM EDTA).
   After microinjection of fertilized oocytes, about 25 injected oocytes were implanted into the oviduct of each surrogate Kunming mouse. The founder mice were identified by genomic PCR three weeks after birth.
   The mouse tail (1cm) was cut off and was digested with TNES (10 mM Tris, pH7.5, 400 mM NaCl, 100 mM EDTA and 0.6% SDS) plus 0.5mg/mL protease K at 56°C for 3 hours. The debris was removed by centrifugation, equal volume of ethanol was added to the supernatant and fully mixed. The precipitated genomic DNA was washed once with 70% ethanol and dried in the air. The dried DNAs were re-suspended in 100µL TE.
   The reaction mixture was prepared by mixing 50 ng DNA template, 200 nM primers, 1 × Taq reaction buffer, 3 mM MgC₂, 200nM dNTP and 2.5 unit Taq DNA polymerase (Sangon, Shanghai); and subjected to genomic PCR amplification. The 478 bp region from CMV promoter to NOK cDNA 5' end was amplified by a pair of primers (P1, P2):
   5'-tggcccgcctggcattatgcccagtacatg-3' (SEQ ID No.1) and
   5'-agccacaggatgaccccaagaaggatgagg-3' (SEQ ID No.2);

   Another pair of primers (P3, P4) were used to amplify a 516 bp DNA fragment from 3' terminus of NOK cDNA to the downstream bovine growth hormone (BGH) poly (A) signal:
   5'-tcctgaagtccctcctaccagcatcctaga-3' (SEQ ID No.3) and
   5'-tcttcccaatcctcccccttgctgtcctgc-3' (SEQ ID No.4).

   After being denatured at 94°C for 5 minutes, the reaction mixture was amplified for 35 cycles at a condition of 94°C for 30s, 50°C for 30s, 72°C, with a PTC 200 Thermal Cycler (MJ Research).
2. The following antibodies used in flow cytometric analysis were all purchased from eBioscience: Phycoerythrin (PE) anti-mouse c-Kit (CD117), PE anti-mouse CD5, Biotin anti-mouse Sca-1 (Ly-6A/E), Fluorescein isothiocyanate (FITC) anti-mouse CD19, FITC anti-mouse Sca-1 (Ly6A/E), Phycoerythrin-Cy7 (PE-Cy7) anti-mouse IgM, FITC anti-mouse CD23, PE anti-mouse CD27, Biotin anti-mouse IgM, Biotin anti-mouse IgD, Allophycocyanin-conjugated streptavidin, FITC streptavidin, PE streptavidin, PE anti-mouse CD43, PE-Cy7 anti-mouse CD45R (B220).

### 3. RNA extraction and reverse transcriptase - polymerase chain reaction (RT-PCR).

Total RNAs were isolated from 100mg tissue using TRIzol Reagent (Invitrogen) according to the instruction. The RNA concentration was determined by absorbance at OD260nm using the spectrophotometer (Amersham pharmacia Biotech). RT-PCR amplification was performed by using the one-step RNA PCR kit (Takara) in a 50µL reaction system. The reaction volume contains: 1µg RNA templates, 5mM MgCl₂, 1mM dNTP, 0.8 unit RNase inhibitor, 0.5 unit AMV RTase XL, 0.5 unit AMV optimized Taq, 400nM each primer plus 1x one-step RNA PCR buffer.

To detect human *NOK* gene expression, a pair of primers was used to amplify a 556bp DNA fragment:
5'-atcggaaagcaggtcct-3' (SEQ ID No.5) and
5'-aagaggctctccactct-3' (SEQ ID No.6) ;

Mouse specific primers were used to amplify a 738bp product of mouse *NOK:*
5'-taatcattgttccggctctc-3' (SEQ ID No.7) and
5'-ccagatgacaaagtttggga-3' (SEQ ID No.8)

To serve as RNA loading control, a pair of primerswere used to amplify the 600bp conserved region between human and mouse actin:
5'-cacactgtgcccatctacga-3' (SEQ ID No.9) and
5'-ctgcttgctgatccacatct-3' (SEQ ID No.10).

The reverse transcription was conducted at 50°C for 20 minutes. After being denatured at 94°C for 2 minutes, the reaction mixture was amplified for 30 cycles at a condition of 94°C for 30s, 50°C for 30s, 72°Cwith a PTC 200 Thermal Cycler (MJ Research).

### 4. Protein extraction and western blot analysis

To analyze NOK protein expression, protein lysates were prepared from lung, liver, spleen, muscle, lymph node and kidney tissues of both wild-type and transgenic mice. About 100mg tissue was chopped into small pieces in 1×PBS ice bath with surgical scissors. Then, the lysis buffer (50mM Tris-HCl, pH 7.5, 150mM NaCl, 1mM EDTA pH 8.0, 0.5% NP40, 1µM pepstatin, 10µM leupeptin and 3µg/mL aprotinin) supplemented with protease inhibitors (1µM PMSF, 100µM Na₃VO₄) was mixed with the chopped tissue.

The prepared samples were resolved onto 10% SDS-PAGE. The sample was incubated with a primary antibody (anti-HA), followed by a secondary antibody conjugated to horseradish peroxidase. The reaction was enhanced and developed by chemiluminescence kit (Pierce).

### 5. Blood smear and staining

The general process to prepare blood smear is as the follows. Place a drop of blood on one end of the slide. Place the second slide as a smear device, with a 30 degree angle on the left side of the blood drop, move the smear device to make the blood drop spread along the edge of the slide. Then, move the smear device quickly from one side of the blood drop to the other, thereby forming on the slide a shape with wing edge. After air drying, fix the smear with methanol. After extensive washing, the blood smear was stained with Wright Giemsa solution at room temperature for 3-5 minutes. For peroxide (POX) staining, a 100:1 mixture of 0.1% tetramethylbenzidine (TMB) and saturated Sodium Nitroprusside was added to the dried smear slide, and stained at room temperature (RT) for 1 minute. Then, 0.7 mL of diluted H₂O₂ was added to the reaction mixture, mixed well, and incubated at room temperature for another 6 minutes. Finally, the reaction mixture was re-stained with Wright-Giemsa solution.

### 6. Histological analysis

After sacrifice, the major organs such as liver, spleen, skeletal muscle, lung, kidney and lymph nodes were isolated from WT and *NOK-tg* mice, and analyzed for tumor cell infiltration. The excised tissues were firstly fixed in 10% formalin, then dehydrated gradually in ethanol, followed by embedding in paraffin, and finally sectioned into paraffin section of 4µm thickness. Glass slides containing the paraffin sections were de-paraffinized in Xylene and rehydrated in diluted ethanol. After stained with hematoxylin and de-stained in acidic alcohol, the sections show blue in bicarbonate and were finally stained with eosin. Also, the paraffin-embedded specimens were sectioned and fixed on a glass slide for immunohistochemistical analysis. The slides were first incubated with rabbit anti-NOK antibody at a dilution of 1:4000 at room temperature for 1 hour. Subsequently, *NOK* gene expression was detected with immunohistochemistry (IHC) polymer detection kit (Zymed).

### 7. Enzyme linked immunosorbent assay (ELISA)

To evaluate the antibody production, sheep red blood cells (SRBC)(53) were used as an antigen to immunize ten 8-12 week old wild type and ten age-matched *NOK-tg* mice (finally six animals for each group were selected for ELISA).

For primary immunization, 1x10⁸ SRBC cells were injected intraperitoneally (i.p.)_into each WT and *NOK*-tg mouse. Serum sample was taken from each immunized mouse after 1, 2, 3 and 4 weeks post-immunization. At the fourth week after primary immunization, mouse was boosted with the second dose of SRBC (1x10⁸). Sera were taken at one week after the secondary SRBC immunization. The titer of immunoglobulin and its variants in the serum of NOK-tg mice was determined by alkaline phosphatase (AP) based enzyme-linked immunosorbent assay (ELISA).

To compare the antibody level of primary humoral immune response in WT and *NOK*-tg mice, serum samples before and after one week SRBC immunization were evaluated for the production of SRBC specific antibodies. The collected sera after primary and secondary immunizations were also quantified for the presence of SRBC-specific antibodies of Ig isotype and their variants.

First, the 96 well ELISA plate was coated with 5 µg Goat anti mouse Ig capture antibody or 5 × 10⁵ sheep red blood cells (SRBC) in 100 µL 1 × PBS, and incubated overnight at 4°C.

On the second day, the coated ELISA plates were washed three times with 0.05% Tween (PBST) in 1 × PBS, and blocked with 1% bovine serum albumin (BSA) at 25 °C for one hour. After washing, about 100 µL diluted antibody (1:1000 dilution) was added into each well, and incubated at 4 °C overnight.

On the third day, the plate was washed for three times using PBS, each well was added with 100µL diluted antibody (AP labeled Goat anti mouse Ig isotype, isotype switching variant, heavy chain or light chain antibody diluted at 1:300), and incubated at 25 °C for 1 hour.

Then the p-Nitrophenyl phosphate substrate was dissolved in the substrate buffer (240 µm MgCl₂ · 6H2O and 10% Diethanolamine, pH 9.8) at the final concentration of 1mg/mL. 100 µL was added to each well. OD405 value was measured at either one minute or five minutes after the addition of the substrates.

### 8. Flow cytometric analysis

About 30mg to 50mg tissues (lymph nodes or spleen) isolated from WT and *NOK*-tg mice were minced into small pieces, and re-suspended in 1mL of ice-cold 1xPBS. The minced tissue was ground onto a 40µm metal filter to make single cell suspension. The collected femurs of both NOK-tg and WT mice were cut and washed repeatedly with staining buffer (2% fetal bovine serum and 0.1% sodium azide, in 1xPBS) to harvest bone marrow (BM) cells. BM single cell suspension was also passed through a 40µm cell filter to removes any cell debris. Peripheral blood mononuclear cells (PMBCs) were purified from the tail blood of WT and *NOK-tg* mice by using Lympholyte®-Mammal purification kit (Cedarlane). The fluorochrome-labelled antibodies were added to an 80µl-staining buffer containing 5x10⁶ cells to be assayed. The reaction mixture was then incubated at 4°C for 30 min, washed three times with staining buffer, and re-suspended into 150µl of OptiLyse®C (Beckman Coulter). For Biotin-conjugated antibody labeling, Allophycocyanin-conjugated streptavidin was added to the reaction mixture. All reaction products were subsequently detected by flow analysis with FACSAria™ flow cytometer (BD Biosciemces).

The enrichment of lineage negative cells from mouse bone marrow was conducted using mouse hematopoietic cell lineage depletion kit (R&D Systems), according to the manual instruction. About 1 x 10⁸ BM cells were incubated with 100 µL MagCellect mouse cell lineage depletion biotinylated antibody mixtures (Rat anti mouse CD5, CD11b, CD45R (B220), Ly-6G (Gr-1) and TER-119) 4 °C for 20 minutes. At the end of incubation, 150 µL of MagCellect streptavidin Magnetic fluid was added and incubated at 4 °C for another 20 minutes. Biotinylated bone marrow cells (lineage positive cells) were isolated from unlabeled cells by using magnets. The lineage negative BM cells were then collected for subsequent analysis

### Results

### 1. Generation and expression analysis of NOK-transgenic mice

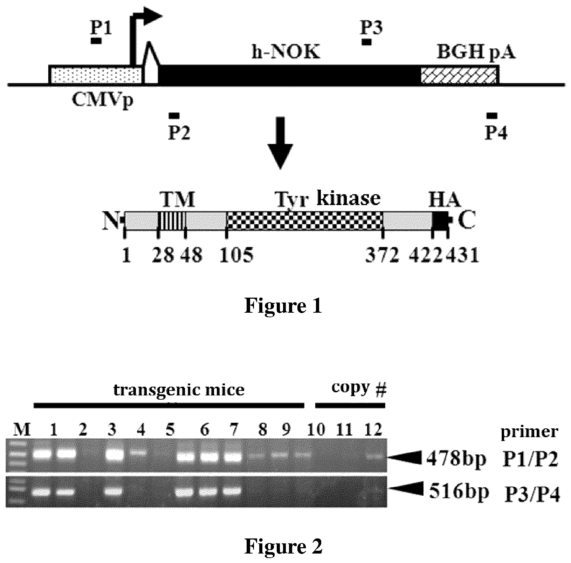

Genomic PCR identified human *NOK* (hNOK) expression cassette has been integrated into Kunming mouse genome (Figure 1 and Figure 2). Four independent strains of *NOK-tg* mice were established.

The *NOK*-tg founder mice strains were mated with wild type of Kunming mice, and then the F1 generation of *NOK*-tg mice were maintained by brother-sister mating to propagate for at least five generations to ensure the stable genetic transmission of NOK in the progeny, and thus *NOK*-tg mice was successfully established.

Therefore, in the context of this application, the NOK transgenic mice (animal models) shall be interpreted as the founder strains well as its progeny having stable inheritance of the NOK transgenic gene.

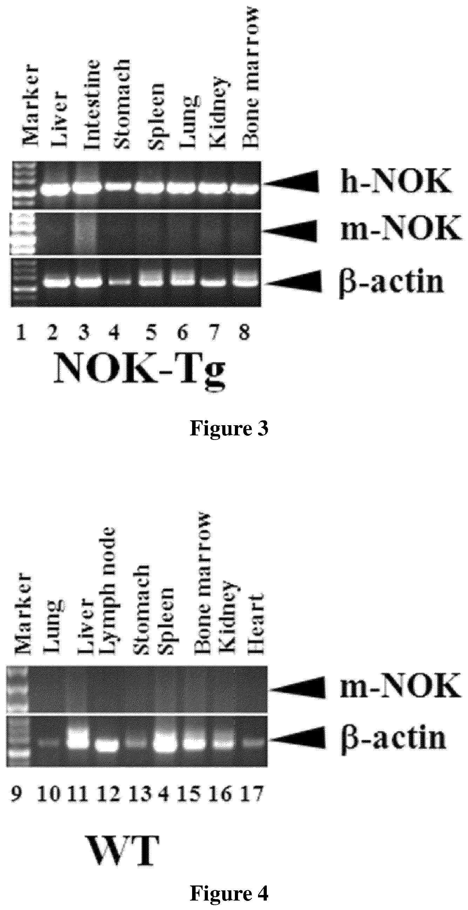

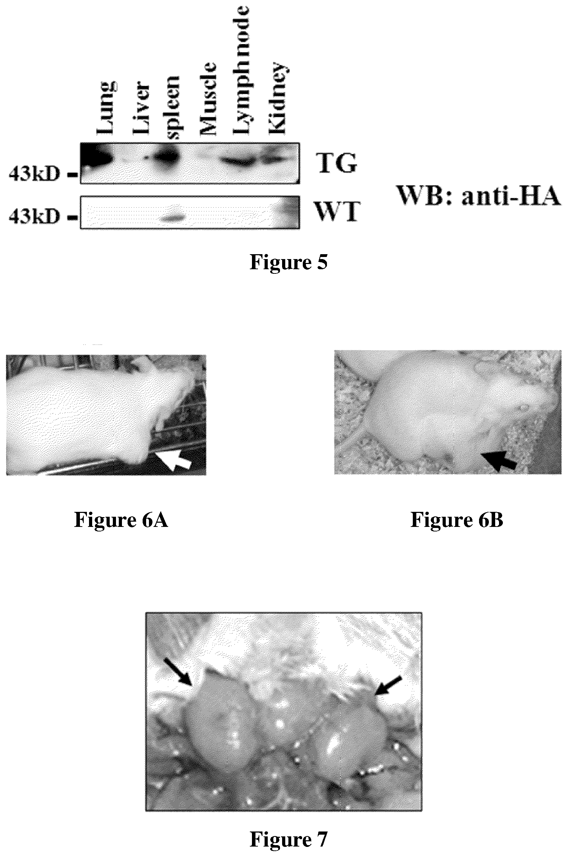

RT-PCR analysis showed the high level of the human *NOK* mRNA but not the mouse *NOK* mRNA expression in all tissues of *NOK-tg* mice (Figure 3). No detectable mouse NOK mRNAs were uncovered from the tissues of wild type littermates (Figure 4). Western blot analysis further confirmed the high level of h*NOK* gene expression in *NOK*-tg mice (Figure 5).

### 2. NOK transgenic mice spontaneously developed lymphocytic malignancy

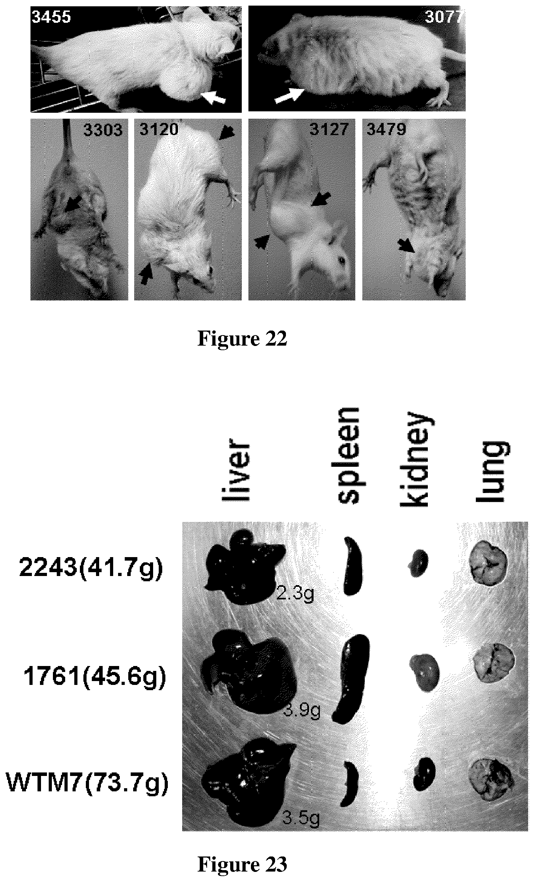

*NOK-tg* mice developed lymphadenectasis featured by the enlargement of lymph nodes at neck, axillary, or inguinal (Figure 6A, 6B). Lymphadenectasis usually initiated at the neck or on one side of the axillary/inguinal; and was observed in about 15% *NOK-tg* mice (Figure 6A, 6B; and Figure 22). These animals often had typical symptoms resembled to human B-CLL such as weight loss, fatigue, itching and bleeding points on skin. Although there was large individual difference, anatomical analysis showed the frequent enlargement of major organs such as liver, spleen, kidney and lung, which can be confirmed according to the weight of the relevant organs (Figure 7, Figure 23).

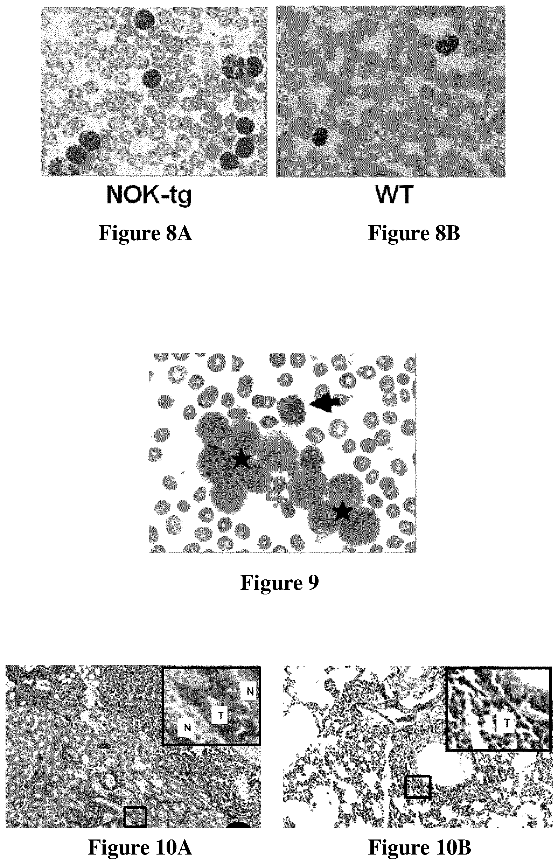

In the animals with lymphadenectasis, blood smear analysis often showed the expanded population of mature lymphocytes (Figure 8A and 8B). To further confirm the result, blood peroxidase (Pox) staining was performed by the inventors, to distinguish malignant cells derived from lymphocytic lineage from those derived from myeloid-mono lineage. Figure 9 showed that these leukemia cells were Pox negative, indicating that the cells were likely to show lymphocytic features.

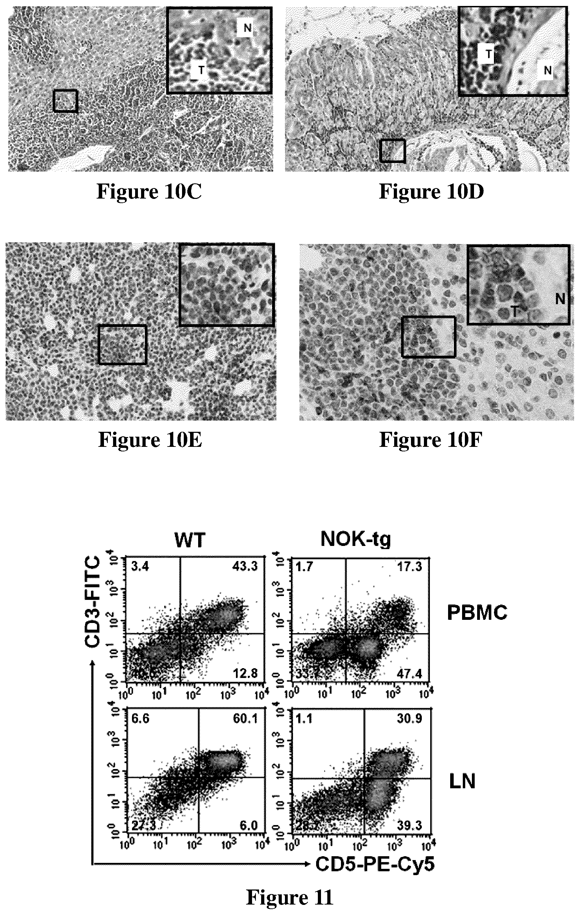

Hematoxylin and eosin staining revealed the infiltrations of tumor cells with mature B lymphocyte appearance in several major organs like kidney, lung, liver and intestine (Figure 10A to 10D). Moreover, immunohistochemistical analysis further demonstrated the positive expression of NOK proteins in the tumor cell infiltrated tissues such as lung and liver sections (Figure 10E and 10F).

Overall, the results strongly indicate that the NOK could induce lymphocytic malignancy in *NOK-tg* mice.

### 3. NOK induced B-CLL

To clarify which cell type was involved in *NOK* induced malignancy, two-color flow cytometric analysis was performed on the cell suspensions prepared from both peripheral blood mononuclear cells (PBMCs) and peripheral lymph nodes with FITC anti-CD3 and PE-Cy5 anti-CD5 antibodies. All selected transgenic mice developed lymphadenectasis. Age-matched wild type mice served as controls. Figure 11 demonstrated that a subpopulation of CD3⁻CD5⁺ lymphocytes was greatly expanded in the peripheral blood (47.4% versus 12.8%) and lymph node (39.3% versus 3%) of *NOK-tg* mice, when compared with wild type mice. Since CD3 is a pan-T cell marker, the expanded CD3⁻CD5⁺ cell subpopulation should not belong to T cell lineage. To further characterize the cell type that NOK might affect, CD19 (a B cell marker) together with CD5 were used in a two-color flow cytometric analysis.

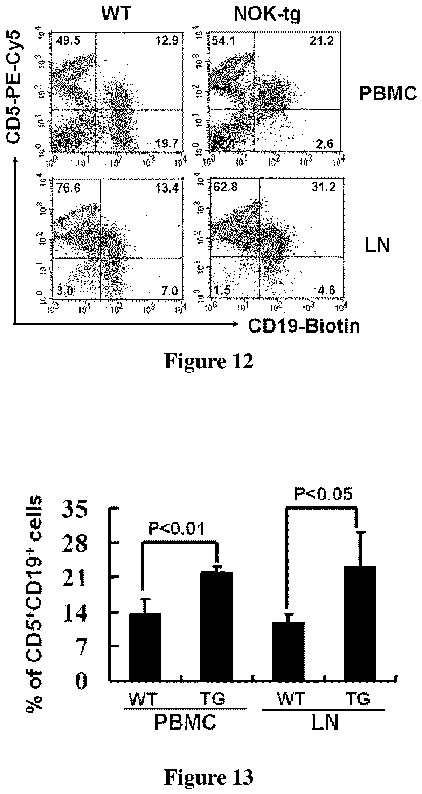

Flow results from Figure 12 and 13 showed that the selected *NOK-tg* mice with enlarged lymph nodes had expanded CD5⁺CD19⁺ subpopulations in both PBMC and peripheral lymph nodes. Since the expanded CD5⁺CD19⁺ phenotype is usually the hallmark of human B-CLL (30), the disease induced in *NOK-tg* mice might be B-CLL.
To evaluate the function of B lymphocyte, the production of immunoglobin (Ig) in wild type and *NOK-tg* mice were measured by standard ELISA test. Except IgM and IgG3, the overall levels of serum IgG1, IgG2a, IgG2b, IgA, κ and λ in *NOK*-tg mice were higher than those in wild type control (Figure 14A). This indicated that, like human B-CLL, B lymphocytic leukemia cells likely to overproduce both Ig isotype and their switching variants (31), indicating that Ig class switch recombination (CSR) may occur. To assess the production of antigen specific antibody, sera were collected from *NOK-tg* and WT mice at two time points, i.e. immediately one-week before (Figure 14B) or one-week after (Figure 14C) SRBC challenge. SRBC-specific IgG1, IgG2a and IgG3 were significantly reduced as compared with that of wild type mice.

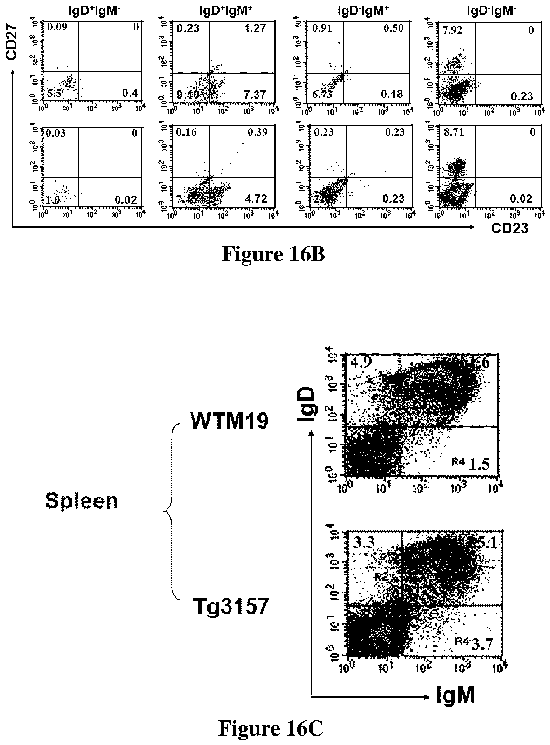

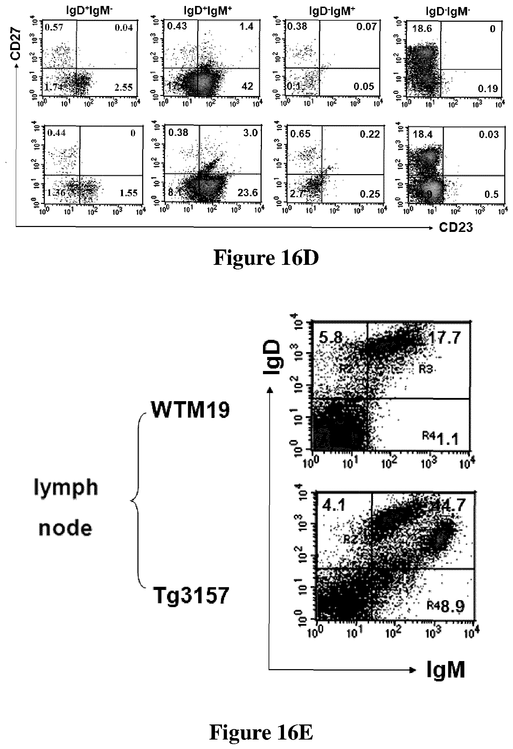

Although being generally at a reduced level in *NOK-tg* mice, there is no statistically significant difference observed in the primary Ig production for SRBC specific IgM, IgA, IgG2a and IgG2b. Interestingly, after secondary boost, the activation level of memory B lymphocytes in *NOK-tg* mice were markedly higher than that of the wild type mice (Figure 15A to 15F), indicating that these tumor cells might have strong memory B cell characters.
In addition to the clonal expansion of CD5⁺CD19⁺ B lymphocytes, human B-CLL usually exhibits low expression level of surface IgM and IgD, and can produce antigen-experienced memory B lymphocytes (3, 4). It has been shown that CD27 is a defined cell surface marker for human memory B lymphocyte (32, 33). Based on the differential expression of IgM, IgD and CD27, human memory B cells can be divided into four groups: IgM⁺IgD⁺CD27⁺, IgM⁻IgD⁻CD27⁺, IgM⁻IgD⁺CD27⁺ and IgM⁺IgD⁻CD27⁺ (33). CD23, as an activated B cell marker, has been shown to be up-regulated in human B-CLL (2, 13). To examine the level of memory B cells in *NOK-tg* mice, four-color flow cytometric analysis on peripheral blood and lymphoid tissues was conducted by using PE-Cy7 anti-IgM, Biotin anti-IgD, PE anti-CD27 and FITC anti-CD23. Results in Figure 16A to 16F showed the great differences of the memory B cells in diverse cellular compartments. In the blood and spleen, the proportions of IgM⁺IgD⁺B lymphocytes were markedly decreased (Figure 16A and 16B, 16C and 16D). Although there was a slightly increase in IgM⁺IgD⁺CD27⁺CD23⁺ (3% in *NOK-tg* versus 1.4% in WT) memory B cells in spleen, no significant increase was observed for other memory B cell subgroups in both spleen and blood (Figure 16A and 16B, 16C and 16D).

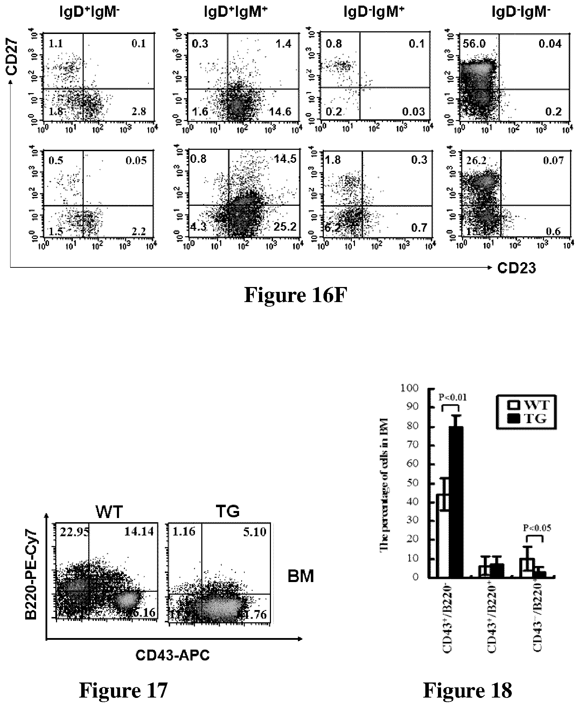

In contrast, a dramatic increase in IgM⁺IgD⁺B lymphocytes was observed in the lymph nodes of *NOK*-tg mice (Figure 16E and 16F). This increase corresponds to an increase of more than 10-fold in IgM⁺IgD⁺CD27⁺CD23⁺ memory B cells in the lymph nodes (Figure 16E and 16F). Thus, the results strongly indicate that the immunophenotypes of *NOK*-tg mice are greatly resembled to that of human B-CLL.

### 4. the immunophenotype heterogenecity of NOK-induced B-CLL in the peripheral lymphoid organs

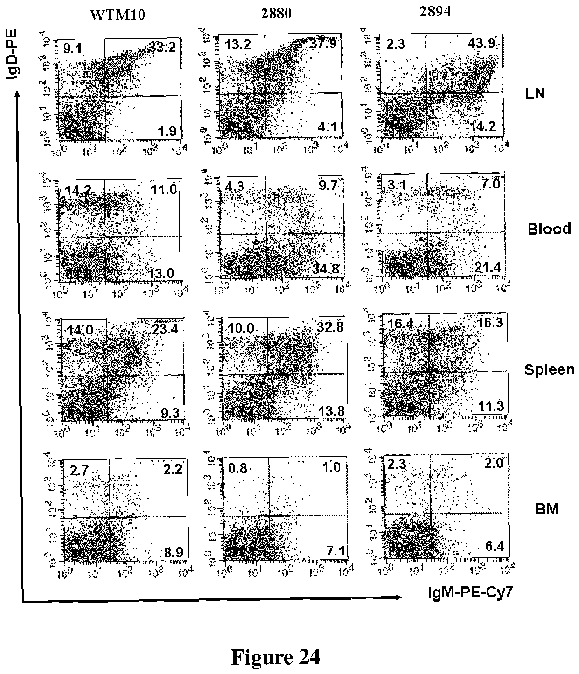

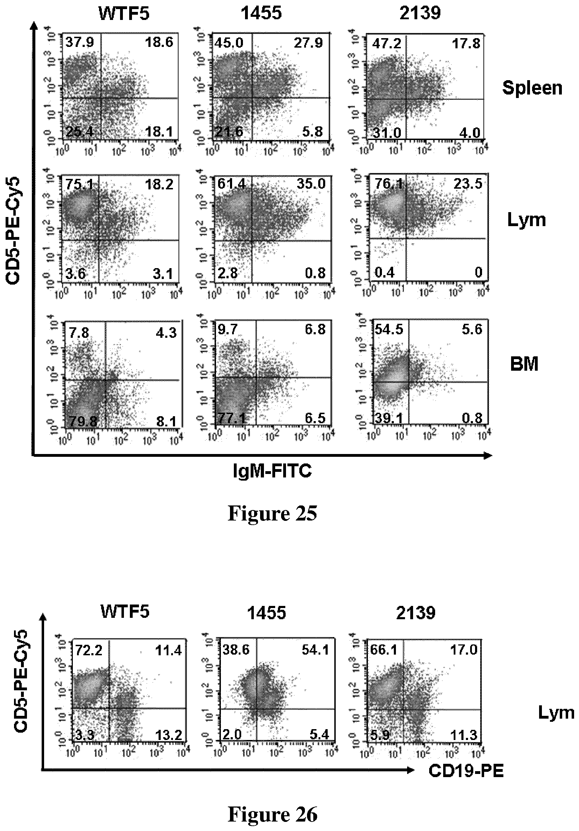

In agreement with human B-CLL (34), the symptoms and the immunophenotypes of *NOK*-induced B-CLL also varied greatly, indicating that the disease progression in *NOK*-tg mice might be dynamic. Consistent with previous observations, IgD⁺/IgM⁺ naive B cell populations were often increased in the enlarged lymph nodes of *NOK*-tg mice. This was in contrast with the decrease of IgD⁺/IgM⁺ naive B lymphocyte in peripheral blood and BM compartments (Figure 24). However, in the peripheral blood but not BM compartment, the population of IgD⁻/IgM⁺ mature B lymphocytes was always markedly expanded (Figure 24). Although an increased IgD⁻IgM⁺ mature B lymphocyte was often found in the spleen of *NOK*-tg mice, comparable increase in IgD⁺/IgM⁺ naive B lymphocytes was not detected. To further analyze the immunophenotype diversities of *NOK*-tg mice, different cell surface markers were chosen. Analysis on IgM and CD5 co-expression also indicates that IgM⁺CD5⁺ double positive cells were commonly expanded in the lymph nodes and slightly increased in BMs, but not always accumulated in the spleens (Figure 25). In agreement with previous results, the expanded CD5⁺/CD19⁺ double positive cells could be detected in the lymph nodes, and reduced IgD⁺/IgM⁺ double positive cells could be detected in BM compartments, respectively (Figure 26 and 27). No direct correlation was observed between the proportions of the immature BP-1⁻/CD24⁺ B cells in BM and that of IgD⁺/IgM⁺ or CD5⁺/CD19⁺ B cells in the peripheral lymphoid organs (Figure 25, 26 and 28), indicating that alterations in immunophenotype might occur dynamically during disease progression in *NOK-tg* animals.

### 5. Suppression of B lymphocyte in BM of NOK-tg mice

To address whether there was a maturational defect during B lymphocyte development in *NOK-tg* mice, the six stages of B cell differentiation in BM (defined by Hardy et al) were analyzed. Four color cytometric analysis using fluorescently-labeled antibodies specific for surface markers such as B220, CD43, BP-1 and CD24 (or IgD and IgM) was conducted to determine the six stages. At least 2.5 to 3.5 fold increase in the most immature stage A was often observed in *NOK-tg* mice, whereas maturation defects were frequently occurred in later stages C to F (Figure 29A to 29C). Regardless of lymphadenectasis, *NOK-tg* mice always have the accumulated B220⁻CD43⁺ cells in BMs (Figure 17 & 18). Analysis on CD43 and CD19 expression in lymph nodes indicates that immature CD43⁺CD19⁺ B lymphocytes were markedly expanded (Figure 30 and 31).

Thus, the results strongly suggests that, in BM, *NOK* might restrict B cell differentiation at A stage and promote the accumulation of B220⁻CD43⁺ progenitor cells that might be further proliferated and expanded in the peripheral lymphoid organs.

### 6. NOK may predispose HSC progenitor cells for the development of B-CLL

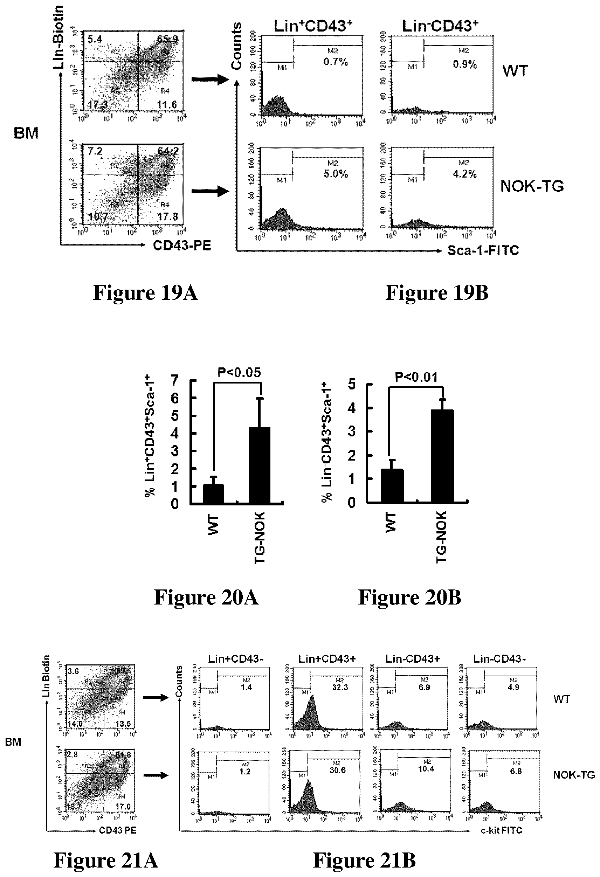

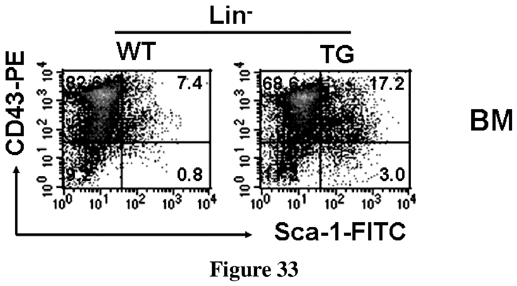

To detect if NOK has direct impact on hematopoietic stem cell development, BM cells were analyzed by using four different antibodies specific for B220, CD43, Sca-1 and c-kit. In transgenic mice, about 3.8 and 5.3 fold increases were observed in B220⁻CD43⁺Sca-1⁺ c-kit⁺ and B220⁻CD43⁺Sca-1⁺c-kit⁻ cell populations, respectively (Figure 32A and 32B). To further analyze the origin of B-CLL, three color fluorescent reagents specific for lineage (Lin) markers, CD43 and Sca-1 were used to determine the potential differences of hematopoietic stem cells in WT and *NOK-tg* mice. A significant increases in both Lin⁺CD43⁺Sca-1⁺ and Lin⁻CD43⁺Sca-1⁺ subpopulations were observed in *NOK-tg* mice (Figure 19A & 19B, Figure 20A & 20B). After depleting the lineage positive cells from BM, it was found that the Lin⁻CD43⁺Sca-1⁺ subpopulation was also markedly expanded (Figure 33). Further, BM cells from WT and *NOK-tg* mice were analyzed for lineage markers, CD43 and c-kit. Consistent with previous results, Figure 21A & 21B demonstrate that the sub-population of Lin⁻CD43⁺c-kit⁺ cells was significantly accumulated in the BM of *NOK-tg* mice. Overall, current data indicates that there is a subpopulation with Lin⁻CD43⁺Sca-1⁺c-kit⁺ surface markers on BM HSC cells of *NOK-tg* mice, these cells might be the progenitor cells to NOK-induced B-CLL.

**Table 1. Comparison of the relative organ weights in WT and NOK-tg mice**

| **Subject*** | **No. of animal** | **Age (weeks)** | **Liver (x10⁻²)^{†}** | | **Spleen (x10⁻³)** | | **Kidney (x10⁻³)** | | **Lung (x10⁻³)** | |
|---|---|---|---|---|---|---|---|---|---|---|
| | | | **Range** | **Mean** | **Range** | **Mean** | **Range** | **Mean** | **Range** | **Mean** 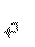 |
| WTF 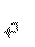 | 8 | 9 ∼ 32 | 4.2 ∼ 5.8 | 5.10 | 2.3 ∼ 4.6 | 3.28 | 5.1 ∼ 6.8 | 5.76 | 4.7 ∼ 6.3 | 5.66 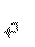 |
| TgF 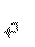 | 11 | 9.5 ∼ 36 | 2.9 ∼ 25.2 | 8.32 | 2.5 ∼ 45.7 | 19.3 | 4.6 ∼ 40.7 | 10.3 | 4.8 ∼ 11.7 | 7.28 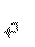 |
| WTM 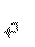 | 8 | 9 ∼ 27 | 4.9 ∼ 8.0 | 6.68 | 2.0 ∼ 4.7 | 2.7 | 4.8 ∼ 9.5 | 7.45 | 3.4 ∼ 6.9 | 4.80 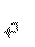 |
| TgM 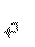 | 14 | 9 ∼ 33 | 3.1 ∼ 17.4 | 5.73 | 1.9 ∼ 50.8 | 8.76 | 4.6 ∼ 22.6 | 13.4 | 4.0 ∼ 13.2 | 5.98 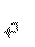 |

| | | | | | | | | | | |
|---|---|---|---|---|---|---|---|---|---|---|
| * Mouse subjects are indicated as wild type female (WTF), wild type male (WTM), transgenic female (TgF), and transgenic male (TgM). † Relative organ weight, calculated as weight of organ divided by whole body weight. | | | | | | | | | | |

### Discussion

B-CLL is the most common leukemia among western population that are mainly caused by the disorder of B lymphocyte in the aged. Although intensive efforts have been carried out to elucidate the pathogenesis of B-CLL, more detailed studies have been hampered by lack of a suitable animal model (39). The inventor demonstrated for the first time that *NOK-tg* mice could spontaneously develop B lymphocyte disease extremely resembled to human B-CLL, which is usually accompanied by superficially enlarged lymph nodes, co-expression of CD5 and CD19 in immunophenotype, and production of tumor cells with memory B cell characteristics. More importantly, the inventors have used this animal model in tracing down the origin of B-CLL and found that NOK gene might direct hematopoietic stem cells toward B-CLL development.

It has been for a long time that people searched for genes associated with B-CLL (20, 22). Genetic and epidemiologic studies suggested that familial CLL might involve gene(s) with pleiotropic character (21, 40) . However, to date no gene(s) associated with CLL has been discovered. In contrast, this study strongly indicates that NOK gene might be the candidate gene eligible for disease predisposition. About 16-35% of human B-CLL patients carry the cytogenetic defect of trisomy 12 (25, 26) . Notably, NOK was initially identified as a novel oncogene from human chromosomal 12, and had great potential to induce tumorgenesis and metastasis in animals (28, 29) . In addition, the NOK expression cassette carried by NOK-tg transgenic mouse genome could be stably delivered and genetically transmitted from one generation to another.

According to the hypothesis of cancer stem cell, only a minor portion of cancer cells with stem cell characters is able to undergo self-renewal (41) . In current study, the inventor found that NOK could promote the expansion of a small portion of primitive stem cells in BM. This type of primitive stem cells showing Lin⁻CD43⁺Sca-1⁺c-kit⁺ immunophenotype is capable of indefinite self-renewal, and undergoes differentiation toward B lymphocyte. It has been demonstrated that the up-regulation of Sca-1 is associated with many types of cancers and plays a positive role in the renewal of cancer stem cells (42) . In another aspect, Sca-1 can function as a negative regulator of T cell proliferation since overexpression of Sca-1 in T cells blocks cellular proliferation (43) . In contrast, deletion of Sca-1 by gene targeting in transgenic mice leads to T cell hyper-proliferation (44) . Mistimed expression of Sca-1 in immature T cells usually results in the impairment of T cell differentiation (45). Thus, NOK-induced up-regulation of Sca-1 in primitive stem cells might be one of the critical elements for the development of B-CLL. Moreover, c-Kit is important for HSC development, HSC deposition to bone marrow 'niche' as well as lymphopoiesis (46, 47). Expression of c-Kit in NOK-induced cancer stem cells might be important for the self-renewal to create a suitable microenvironment for B-CLL formation. Co-expression of CD43 with other markers has been found in human B-CLL (48, 49), and inclusion of this molecule could help the diagnosis of B-CLL (50). Consistently, the results herein demonstrated that the up-regulation of CD43 expression might be one of the key characters of NOK-induced B-CLL. The increased CD43⁺ cell populations in NOK-tg mice can be detected in different cellular compartments from central lymphoid bone marrow to the peripheral lymph nodes. It is believed that CD43 expression on B cell surface promotes B cell proliferation and survival (51) . Therefore, the increased CD43 expression observed in NOK-tg mice may have a great impact on the malignancy of NOK-induced B-CLL.

### References

1. Chiorazzi, N., Rai, K.R. and Ferrarini, M. 2005. N Engl J Med 352:804-815.
2. Dighiero, G. and Hamblin, T.J. 2008. Lancet 371:1017-1029.
3. Ghia, P et al.,2007. Crit Rev Oncol Hematol 64:234-246.
4. Rozman, C. and Montserrat, E. 1995. N Engl J Med 333:1052-1057.
5. Hamblin, T.J et al.,1999. Blood 94:1848-1854.
6. Krober, A. et al., 2002. Blood 100:1410-1416.
7. Messmer, B.T et al.,2004. Blood 103:3490-3495.
8. Klein, U. et al.,2001. J Exp Med 194:1625-1638.
9. Rosenwald, A et al.,2001. J Exp Med 194:1639-1647.
10. Lanham, S. et al., 2003. Blood 101:1087-1093.
11. Chen, L. et al.,2005. Blood 105:2036-2041.
12. Cutrona, G. et al.,2008. Haematologica 93:413-422.
13. Damle, R.N. et al.,2002. Blood 99:4087-4093.
14. Damle, R.N et al.,1999. Blood 94:1840-1847.
15. Wiestner, A et al.,2003. Blood 101:4944-4951.
16. Chen, L et al.,2002. Blood 100:4609-4614.
17. Stevenson, F.K. and Caligaris-Cappio, F. 2004. Blood 103:4389-4395.
18. Boggs, D.R. et al.,1987. Am J Hematol 25:349-354.
19. Goldin, L.R. and Slager, S.L. 2007. Hematology Am Soc Hematol Educ Program 2007:339-345.
20. Sellick, G.S et al.,2006. Semin Oncol 33:195-201.
21. Goldin, L.R., Pfeiffer, R.M., Li, X. and Hemminki, K. 2004. Blood 104:1850-1854.
22. Plass, C. et al.,2007. Br J Haematol 139:744-752.
23. Liu, Y. et al.,1997. Oncogene 15:2463-2473.
24. Gaidano, G et al.,2012. J Clin Invest 122:3432-3438.
25. Escudier, S.M et al.,. 1993. Blood 81:2702-2707.
26. Que, T.H et al.,1993. Blood 82:571-575.
27. Cosson, A et al.,2014. Genes Chromosomes Cancer 53:657-666.
28. Chen, Y. et al.,2005. Cancer Res 65:10838-10846.
29. Liu, L. et al.,2004. Cancer Res 64:3491-3499.
30. Matutes, E. et al.,1994. Leukemia 8:1640-1645.
31. Lanasa, M.C. and Weinberg, J.B. 2011. Leuk Lymphoma 52(7):1398-1400.
32. Agematsu, K et al.,2000. Immunol Today 21:204-206.
33. Klein, U et al.,1998. J Exp Med 188:1679-1689.
34. Chiorazzi, N. and Ferrarini, M. 2006. Hematology Am Soc Hematol Educ Program:273-278, 512.
35. Hunte, B.E. et al.,1998. Eur J Immunol 28:3850-3856.
36. Wu, Q et al.,1989. J Immunol 143:3303-3308.
37. Hardy, R.R et al.,1991. J Exp Med 173:1213-1225.
38. Li, Y.S., Hayakawa, K. and Hardy, R.R. 1993. J Exp Med 178:951-960.
39. Michie, A.M et al.,2007. Biochem Soc Trans 35:1009-1012.
40. Caporaso, N et al.,2007. Br J Haematol 139:630-634.
41. Krause, D.S. and Van Etten, R.A. 2007. Trends Mol Med 13:470-481.
42. Holmes, C. and Stanford, W.L. 2007. Stem Cells 25:1339-1347.
43. Henderson, S.C., Kamdar, M.M. and Bamezai, A. 2002. J Immunol 168:118-126.
44. Stanford, W.L et al.,1997. J Exp Med 186:705-717.
45. Bamezai, A et al.,1995. J Immunol 154:4233-4239.
46. Driessen, R.L., Johnston, H.M. and Nilsson, S.K. 2003. Exp Hematol 31: 1284-1291.
47. Waskow, C et al.,2002. Immunity 17:277-288.
48. Quijano, S et al.,2008. Cytometry B Clin Cytom 74:139-149.
49. Rolinski, J et al.,1999. Pol Arch Med Wewn 102:753-762.
50. Jung, G et al.,2003. Br J Haematol 120:496-499.
51. Dragone, L.L et al.,1995. Proc Natl Acad Sci U S A 92:626-630.
52. Yang, G et al.,2005. Genesis 42:33-36.
53. Inouye, S et al.,2004. J Biol Chem 279:38701-38709.

## Claims

1. Use of NOK gene and of the expression product thereof in the construction of animal model of chronic B lymphocytic leukemia.

2. Use according to claim 1, wherein:
the chronic lymphocytic leukemia is chronic B lymphocytic leukemia;
preferably, mutant chronic B lymphocytic leukemia, or unmutated chronic B lymphocytic leukemia.

3. Use according to claim 1, wherein the animal is a mammal other than human; and the animal is selected from the group consisting of: mouse, rat, guinea pig, rabbit, bovidae, caprid, equidae, camelid, suidae, canidae, felidae, monkey, ape;
preferably, the animal is mouse.

4. Use according to claim 1, wherein said animal model shows any one symptom or a combination thereof selected from the group consisting of weight loss, fatigue, itching, bleeding points on skin, organ enlargement, invasion of tumor cells into organ;
the organ is selected from the group consisting of: liver, spleen, kidney, lung, lymph node, peripheral blood, bone marrow, or combination thereof.

5. Use according to claim 1, wherein the expression product is selected from the group consisting of: mRNA or complementary sequence thereof, cDNA or complementary sequence thereof, NOK mature protein, NOK precursor protein, or fragment, mutant, derivative or modified product of any of the above;
the modification is selected from the group consisting of: phosphorylation, glycosylation, acetylation, pegylation, or combination thereof.

6. Use according to claim 1, wherein the NOK gene is represented by Genbank accession number KP729000.1.

7. A method for constructing an animal model of chronic B lymphocytic leukemia, comprising:
1) providing an expression vector expressing NOK,
2) introducing the expression vector into oocyte of animal, preferably by microinjection;
3) transferring the oocyte injected with the expression vector to surrogate animal for growth;
4) NOK transgenic animal(s) is/are produced by the surrogate animal;
the animal model shows any one symptom or a combination thereof selected from the group consisting of weight loss, fatigue, itching, bleeding points on skin, organ enlargement, invasion of tumor cells into organ;
the organ is selected from the group consisting of: liver, spleen, kidney, lung, lymph node, peripheral blood, bone marrow, or combination thereof.

8. The method according to claim 7, wherein said chronic lymphocytic leukemia is chronic B-lymphocytic leukemia; preferably mutant chronic B-lymphocytic leukemia, or unmutated chronic B-lymphocytic leukemia.

9. The method according to claim 7, wherein the animal is mammal other than human; and the animal is selected from the group consisting of mouse, rat, guinea pig, rabbit, bovidae, caprid, equidae, camelid, suidae, canidae, felidae, monkey, ape;
preferably, the animal is mouse.

10. A method for constructing a mouse animal model of chronic B lymphocytic leukemia, comprising:
1) providing an expression vector expressing human NOK,
2) introducing the expression vector into mouse oocytes, preferably by microinjection;
3) transferring the oocyte injected with the expression vector to surrogate mouse for growth;
4) NOK transgenic mouse/mice is/are produced by the surrogate mouse; optionally, the transgenic mouse further comprises progeny thereof that expresses human NOK.
